# EUROPÄISCHE PATENTANMELDUNG

(11) **EP 4 703 478 A1**
(43) Veröffentlichungstag der Anmeldung: **04.03.2026**
(21) Anmeldenummer: 24196609.2
(22) Anmeldetag: 27.08.2024
(51) Int. Cl.: C12Q 1/56, G01N 33/86

(54) **VERFAHREN ZUM AUSSCHLUSS VON FAKTOR XIA-INHIBITOREN IN PATIENTENPROBEN**

(71) Anmelder: Siemens Healthcare Diagnostics Products GmbH, 35041 Marburg (DE)
(72) Erfinder: Zander, Norbert, 35039 Marburg (DE); Duwe, Christa, 35075 Gladenbach (DE); Naumann, Katharina, 35037 Marburg (DE)
(74) Vertreter: Siemens Healthineers Patent Attorneys

(57) **Zusammenfassung**

Die vorliegende Erfindung liegt auf dem Gebiet der Gerinnungsdiagnostik und betrifft ein Verfahren zum Ausschluss von Antikoagulanzien aus der Gruppe der Faktor XIa-Inhibitoren in einer Patientenprobe.

## Beschreibung

Die vorliegende Erfindung liegt auf dem Gebiet der Gerinnungsdiagnostik und betrifft ein Verfahren zum Ausschluss von Antikoagulanzien aus der Gruppe der Faktor XIa-Inhibitoren in einer Patientenprobe.

Das Blutgerinnungssystem (Hämostase) erfüllt die Aufgabe, einerseits eine kontinuierliche Strömung des Blutes zu gewährleisten und anderseits im Falle einer Verletzung rasch und lokal einen Wundverschluss herbeizuführen, um einen Blutverlust zu minimieren. Die Fibrinbildung, die zur Bildung der wundverschließenden Blutgerinnsel beiträgt, steht am Ende einer Kaskade enzymatischer Prozesse, wobei jedes Enzym ein weiteres Enzym durch proteolytische Prozesse aktiviert. Beispiele für solche prokoagulatorischen Enzyme sind Thrombin (Faktor IIa), Faktor Xa und Faktor XIa. Sowohl bei Thrombin als auch bei Faktor Xa und Faktor XIa handelt es sich um Serinproteasen, die normalerweise in Form von inaktiven Proenzymen im Blut vorkommen und erst im Zuge der Gerinnungsaktivierung aktiviert werden.

Faktor XI ist ein Bestandteil des intrinsischen Systems der Blutgerinnung und wird durch den aktivierten Faktor XII oder durch Thrombin aktiviert und aktiviert seinerseits den Faktor IX, der dann über die Aktivierung von Faktor X schließlich die Thrombinbildung beeinflusst.

In der Antikoagulationstherapie werden zunehmend neue direkte Gerinnungsfaktorinhibitoren, insbesondere direkte Thrombin- und Faktor Xa-Inhibitoren und seit Neuestem auch Faktor XI-Inhibitoren eingesetzt. Diese neuen Gerinnungshemmer haben das Potenzial, die bislang benutzten indirekten Gerinnungshemmer, vor allem Heparin und dessen Derivate, die ihre gerinnungshemmende Wirkung nur im Zusammenspiel mit Kofaktoren wie Antithrombin oder Heparin Cofaktor II entfalten, abzulösen. Für die Steuerung der Therapie und die Dosierung der Medikamente ist es daher wichtig, die Menge des therapeutischen Inhibitors zu kennen. Es ist daher notwendig, über diagnostische Methoden zu verfügen, die es ermöglichen, die Anwesenheit, die Wirksamkeit oder die Plasmakonzentrationen der therapeutischen Gerinnungsfaktorinhibitoren zu bestimmen.

Im Stand der Technik sind verschiedene Testverfahren zum quantitativen Nachweis von direkten Inhibitoren von proteolytisch wirksamen Gerinnungsfaktoren, wie z.B. Inhibitoren von Thrombin, Faktor Xa oder FXIa, in humanen Plasmaproben bekannt.

Die Bestimmung von Inhibitoren von proteolytisch wirksamen Gerinnungsfaktoren erfolgt überlicherweise mit Hilfe chromogener oder koagulometrischer Testverfahren. Bei den chromogenen Verfahren wird die Patientenprobe, die z.B. einen Thrombin- bzw. Faktor Xa-Inhibitor vermutlich enthält, mit einer sich im Überschuss befindlichen, definierten Menge des entsprechenden aktivierten Gerinnungsfaktors und einem chromogenen Substrat für den aktivierten Gerinnungsfaktor vermischt, und die im Reaktionsansatz verbleibende Aktivität des Gerinnungsfaktors wird photometrisch gemessen. Bei den etablierten, auch kommerziell erhältlichen chromogenen Testen werden insbesondere die Chromophore para-Nitroanilin (pNA) und 5-Amino-2-Nitro-Benzoesäure (ANBA), welche ein Absorptionsmaximum bei 405 nm aufweisen, verwendet. Die entstehende gelbe Farbe wird in der Regel photometrisch bestimmt. Je höher die Konzentration des therapeutischen Inhibitors in der Patientenprobe ist, desto mehr wird die Aktivität des zugegebenen Gerinnungsfaktors gehemmt und desto weniger Substrat wird gespalten. Bei der Bestimmung von Inhibitoren verhält sich die Farbkonzentration im Testansatz umgekehrt proportional zur Inhibitorkonzentration in der Probe. Beispielsweise in EP-B1-0034320 oder in EP-A2-0004271 sind derartige Thrombin- bzw. Faktor Xa-basierte, chromogene Testverfahren beschrieben. Mit Hilfe dieser Teste können therapeutische Inhibitoren, die die Aktivität von Blutgerinnungsfaktoren inhibieren, in Patientenproben quantitativ bestimmt werden. In der WO-A1-2012069139 ist ein Verfahren zur Bestimmung von Faktor Xa-Inhibitoren und in der WO-A1-2012175183 ein Verfahren zur Bestimmung von Thrombininhibitoren in Serum- und Urinproben beschrieben.

Mit der zunehmenden Anwendung unterschiedlichster Gerinnungsfaktorinhibitoren zur Behandlung oder Prophylaxe thrombotischer Erkrankungen entsteht eine wachsende Notwendigkeit schnelle und einfache diagnostische Verfahren bereitzustellen, die es ermöglichen festzustellen, ob ein Patient, der beispielsweise bewusstlos in ein Krankenhaus eingeliefert wird und über dessen Vorerkrankungen oder Medikamenteneinnahme keine Informationen vorliegen, mit Antikoagulanzien behandelt wird und wenn ja mit welchen. Insbesondere im Fall dringend durchzuführender operativer Eingriffe ist es für die Einschätzung des Risikos von Blutungskomplikationen wichtig, den Blutgerinnungsstatus eines Patienten und eine etwaige Antikoagulanztherapie zu kennen.

In diesem Zusammenhang ist es wünschenswert über einen einfachen und schnellen Test zu verfügen, mit dem die Anwesenheit eines Antikaogulanzes aus der Gruppe der Faktor XIa-Inhibitoren in einer Patientenprobe ausgeschlossen werden kann.

Der vorliegenden Erfindung lag daher die Aufgabe zugrunde, ein einfaches Verfahren zum Ausschluss von Antikoagulanzien aus der Gruppe der Faktor XIa-Inhibitoren in einer Patientenprobe bereitzustellen, das eine hohe Sensitivität und Spezifität für das Fehlen von Faktor XIa-Inhibitoren aufweist.

Die Aufgabe wird dadurch gelöst, dass auf der Basis eines konventionellen APTT-Tests, der typischerweise in jedem klinischen Labor verfügbar ist, ein Verfahren bereitgestellt wird, bei dem zunächst die Patientenprobe und eine Normalplasmaprobe mit einem Faktor XI-Mangelplasma und mit mindestens einer neutralisierenden Substanz, die ein anderes Antikoagulanz als ein Antikoagulanz aus der Gruppe der Faktor XIa-Inhibitoren neutralisiert, vermischt werden und eine Bestimmung der APTT (aktivierte partielle Thromboplastinzeit) in den beiden Proben vorgenommen wird. Die so ermittelten Gerinnungszeiten der beiden Proben werden durch eine Quotientenbildung zueinander ins Verhältnis gesetzt, und der Quotient wird mit einem vorbestimmten Referenzwert verglichen, wobei die Anwesenheit eines Faktor XIa-Inhibitors in der Patientenprobe ausgeschlossen werden kann, wenn der Quotient unterhalb des vorbestimmten Referenzwerts liegt

Gegenstand der Erfindung ist also ein Verfahren zum Ausschluss von Antikoagulanzien aus der Gruppe der Faktor XIa-Inhibitoren in einer Patientenprobe, das Verfahren umfassend die Schritte
a) Herstellen eines ersten Reaktionsgemisches enthaltend die Patientenprobe und eines zweiten Reaktionsgemisches enthaltend eine Normalplasmaprobe durch Vermischen jeder der beiden Proben mit
   i) einem Faktor XI-Mangelplasma, und
   ii) mindestens einer neutralisierenden Substanz, die ein anderes Antikoagulanz als ein Antikoagulanz aus der Gruppe der Faktor XIa-Inhibitoren neutralisiert, und
   iii) einem Gerinnungsaktivator des intrinsischen Blutgerinnungssystems;
b) Messen der Gerinnungszeit in dem ersten Reaktionsgemisch (APTT XI_{Probe}) und der Gerinnungszeit in dem zweiten Reaktionsgemisch (APTT XI_{Normal});
c) Bilden des Quotienten APTT XI_{Probe}/APTT XI_{Normal} aus beiden Gerinnungszeiten;
d) Vergleichen des Quotienten mit einem vorbestimmten Referenzwert; und
e) Ausschließen von Antikoagulanzien aus der Gruppe der Faktor XIa-Inhibitoren in der Patientenprobe, wenn der Quotient APTT XI_{Probe}/ APTT XI_{Normal} unterhalb des vorbestimmten Referenzwerts liegt.

Der Begriff "Antikoagulanzien aus der Gruppe der Faktor XIa-Inhibitoren" bezeichnet eine therapeutisch wirksame Substanz, die natürlicherweise nicht im menschlichen Körper vorkommt und die die proteolytische Aktivität des Gerinnungsfaktors XIa spezifisch, typischerweise durch direkte Bindung an den Faktor XIa (oder XI) reduziert. Der Begriff "Antikoagulanzien aus der Gruppe der Faktor XIa-Inhibitoren" umfasst explizit nicht gerinnungshemmende Proteine oder Proteinkomplexe, die natürlicherweise im menschlichen Körper vorkommen, wie z.B. Antithrombin oder Heparin Cofaktor II, und die direkt oder indirekt die proteolytische Aktivität des Gerinnungsfaktors XIa hemmen können.

Derzeit bekannte Antikoagulanzien aus der Gruppe der Faktor XIa-Inhibitoren sind beispielsweise Asundexian, Milvexian und Abelacimab sowie Osocimab, Gruticibart (Xisomab), REGN9933, MK-2060, ONO-7684 oder ONO-5450598.

Unter einer "Patientenprobe" ist eine Plasmaprobe, z.B. eine Citratplasmaprobe, eines Individuums zu verstehen.

Unter einer "Normalplasmaprobe" ist eine Plasmaprobe eines offensichtlich gesunden Blutspenders oder eine Probe einer Mischung von Plasmen von mehreren, z.B. von 20, offensichtlich gesunden Blutspendern, also eines Normalplasmapools, zu verstehen. In einem Normalplasma liegen die Werte aller Gerinnungsfaktoren im Normalbereich.

Der Begriff "Faktor XI-Mangelplasma" bezeichnet ein humanes oder anderes tierisches Plasma, dem Faktor XI beispielsweise durch Immunadsorption vollständig oder zumindest nahezu entzogen wurde, während alle übrigen Gerinnungsfaktoren in normalen Konzentrationen vorhanden sind. Typischerweise werden Plasmen mehrerer offensichtlich gesunder Blutspender (Mensch oder Tier) zu einem Normalplasmapool gepoolt und dieser Normalplasmapool wird dann durch Adsorption, z.B. unter Verwendung von Faktor XI-spezifischen monoklonalen und/oder polyklonalen Antikörpern, von Faktor XI befreit oder weitestgehend befreit, wodurch ein Faktor XI-Mangelplasma erhalten wird.

Erfindungsgemäß werden die Patientenprobe und die Normalplasmaprobe mit Faktor XI-Mangelplasma vermischt, indem pro Volumenteil Patienten- bzw. Normalplasmaprobe vorzugsweise zwei Volumenteile Faktor XI-Mangelplasma zugegeben werden. Die Verdünnung der Patientenprobe mit Faktor XI-Mangelplasma bewirkt, dass etwaige Gerinnungsfaktormängel in der Patientenprobe ausgeglichen werden und sie damit keinen Einfluss auf die spätere Gerinnungsreaktion haben.

Ferner wird der Patientenprobe und der Normalplasmaprobe mindestens eine neutralisierende Substanz, die ein anderes Antikoagulanz als ein Antikoagulanz aus der Gruppe der Faktor XIa-Inhibitoren neutralisiert, zugegeben. Dies hat den Zweck, dass die Gerinnungszeit-verlängernde Wirkung etwaiger anderer Antikoagulanzien, die in der Patientenprobe enthalten sein könnten, eliminiert wird, sie damit keinen Einfluss auf die spätere Gerinnungsreaktion haben. Welche Antikoagulanzien bevorzugterweise neutralisiert werden sollten, kann je nach Zeit und Ort variieren, da sich im Laufe der Jahre oder in den Gesundheitssystemen verschiedener Länder die Popularität bestimmter Antikoagulanzien unterscheiden kann. Der Patientenprobe und der Normalplasmaprobe können auch mehrere neutralisierende Substanzen, die ein anderes Antikoagulanz oder verschiedene andere Antikoagulanzien als ein Antikoagulanz aus der Gruppe der Faktor XIa-Inhibitoren neutralisieren, zugegeben werden.

Vorzugsweise handelt es sich bei der mindestens einen Antikoagulanz-neutralisierenden Substanz um eine Substanz, die ein Antikoagulanz aus der Gruppe der Thrombin- und FXa-Inhibitoren neutralisiert. Eine geeignete Thrombin-Inhibitoren-neutralisierende Substanz ist beispielweise Idarucizumab; geeignete FXa-Inhibitoren-neutralisierende Substanzen sind beispielsweise Andexanet alfa und Ciraparantag.

Besonders bevorzugterweise handelt es sich bei der mindestens einen Antikoagulanz-neutralisierenden Substanz um eine Substanz, die Heparin neutralisiert. Eine geeignete Heparin-neutralisierende Substanz ist beispielweise Protaminsulfat.

Der mit einem Faktor XI-Mangelplasma und mit mindestens einer neutralisierenden Substanz, die ein anderes Antikoagulanz als ein Antikoagulanz aus der Gruppe der Faktor XIa-Inhibitoren neutralisiert, vermischten Patientenprobe wird dann zur Herstellung eines ersten Reaktionsgemisches ein Gerinnungsaktivator des intrinsischen Blutgerinnungssystems zugegeben. Analog wird der mit einem Faktor XI-Mangelplasma und mit mindestens einer neutralisierenden Substanz, die ein anderes Antikoagulanz als ein Antikoagulanz aus der Gruppe der Faktor XIa-Inhibitoren neutralisiert, vermischten Normalplasmaprobe zur Herstellung eines zweiten Reaktionsgemisches derselbe Gerinnungsaktivator des intrinsischen Blutgerinnungssystems zugegeben.

Ein Gerinnungsaktivator des intrinsischen Blutgerinnungssystems besteht typischerweise aus einer Kombination von Phospholipiden (sogenannte partielle Thromboplastine), Calciumionen und einer oberflächenaktiven Substanz, wie z.B. Kaolin oder Ellagsäure oder Silica etc. Durch die Zugabe eines Gerinnungsaktivator des intrinsischen Blutgerinnungssystems zu einer Blut- oder Plasmaprobe wird die Blutgerinnung im Reaktionsgemisch in Gang gesetzt.

In den beiden Reaktionsgemischen wird dann die Zeit (in Sekunden) bis zum Eintreten der Gerinnselbildung, also die Gerinnungszeit, genauer die aktivierte partielle Thromboplastinzeit (APTT), gemessen. Die Gerinnungszeit kann durch manuelle oder automatische Methoden bestimmt werden. Bei der automatischen Bestimmung ist die Messung einer mechanischen oder einer optischen Eigenschaft des Reaktionsgemisches, z.B. der Viskosität oder Trübung, sehr verbreitet. In allen Fällen automatischer Messung wird eine Eigenschaft des Reaktionsgemisches kontinuierlich gemessen, und aus der zeitabhängigen Änderung der Eigenschaft kann mit Hilfe von herkömmlichen Auswerteverfahren die Gerinnungszeit als Endpunkt bestimmt werden.

Erfindungsgemäß wird die Gerinnungszeit in dem ersten Reaktionsgemisch, welches die Patientenprobe enthält, gemessen (APTT XI_{Probe}), und es wird die Gerinnungszeit in dem zweiten Reaktionsgemisch, welches die Normalplasmaprobe enthält, gemessen (APTT XI_{Normal}). Aus den beiden so ermittelten Gerinnungszeiten wird dann der Quotient APTT XI_{Probe}/APTT XI_{Normal} gebildet, und der ermittelte Quotient wird mit einem vorbestimmten Referenzwert verglichen.

Der Referenzquotient wird üblicherweise vorab bestimmt, typischerweise indem für eine statistisch hinreichende Anzahl von Proben von Patienten, die bekanntermaßen mit einem Antikoagulanz aus der Gruppe der Faktor XIa-Inhibitoren therapiert werden, und von offensichtlich gesunden, unbehandelten Normalspendern und gegebenenfalls von Patienten, die bekanntermaßen mit einem anderen Antikoagulanz als einem Antikoagulanz aus der Gruppe der Faktor XIa-Inhibitoren therapiert werden (z.B. mit Heparin oder einem direkten Thrombin- oder Faktor Xa-Inhibitor), mit dem erfindungsgemäßen, modifizierten APTT-Test (Zugabe von FXI-Mangelplasma und einer Antikoagulanz-neutralisierenden Substanz) die Gerinnungszeit bestimmt wird, die Quotienten der Gerinnungszeiten der Faktor XIa-Inhibitor-positiven Proben und der Faktor XIa-Inhibitor-negativen Proben ermittelt und ins Verhältnis zu der/den Gerinnungszeiten von Normalplasmaproben gesetzt werden. Aus der Menge der ermittelten Quotienten kann dann ein Referenzwert (Referenzquotient, Grenzwert, Cut off-Wert) ermittelt werden, der eine möglichst sichere Unterscheidung zwischen Faktor XIa-Inhibitor-negativen und Faktor XIa-Inhibitor-positiven Patientenproben ermöglicht.

Es wurde gefunden, dass die Anwesenheit eines Antikoagulanzes aus der Gruppe der Faktor XIa-Inhibitoren in der Patientenprobe ausgeschlossen werden kann, wenn der Quotient APTT XI_{Probe}/APTT XI_{Normal} unterhalb des vorbestimmten Referenzwerts liegt.

Ein weiterer Gegenstand der vorliegenden Erfindung ist ein Testkit zur Verwendung in einem Verfahren zum Ausschluss von Antikoagulanzien aus der Gruppe der Faktor XIa-Inhibitoren in einer Patientenprobe, das Testkit enthaltend
a) ein Faktor XI-Mangelplasma, und
b) mindestens eine neutralisierende Substanz, die ein anderes Antikoagulanz als ein Antikoagulanz aus der Gruppe der Faktor XIa-Inhibitoren neutralisiert.

Das Faktor XI-Mangelplasma und die mindestens eine neutralisierende Substanz (beide wie weiter oben beschrieben) können jeweils als separates Reagenz, in flüssiger Form oder als resuspendierbares Lyophilisat, bereitgestellt werden Alternativ kann das Testkit ein Reagenz enthalten, das eine Mischung des Faktor XI-Mangelplasmas und der mindestens einen neutralisierenden Substanz enthält.

Das Reagenz, welches die mindestens eine neutralisierende Substanz enthält, die ein anderes Antikoagulanz als ein Antikoagulanz aus der Gruppe der Faktor XIa-Inhibitoren neutralisiert, egal, ob es sich um ein separates Reagenz oder ein Reagenz zusätzlich enthaltend das Faktor XI-Mangelplasma oder ein Reagenz zusätzlich enthaltend einen Gerinnungsaktivator des intrinsischen Blutgerinnungssystems (siehe weiter unten) handelt, enthält vorzugsweise eine neutralisierende Substanz, die ein Antikoagulanz aus der Gruppe der Thrombin- und FXa-Inhibitoren neutralisiert, wie beispielsweise die Thrombin-Inhibitoren neutralisierende Substanz Idarucizumab und/oder mindestens eine der FXa-Inhibitoren neutralisierenden Substanzen Andexanet alfa und Ciraparantag. Besonders bevorzugterweise ist in dem Reagenz die Heparin-neutralisierende Substanz Protaminsulfat enthalten.

Bevorzugterweise enthält ein erfindungsgemäßes Testkit zusätzlich einen Gerinnungsaktivator des intrinsischen Blutgerinnungssystems. Der Gerinnungsaktivator wird vorzugsweise als separates Reagenz in flüssiger Form oder als resuspendierbares Lyophilisat, bereitgestellt und enthält bevorzugterweise eine Kombination aus Phospholipiden und einem Kontaktaktivator, bevorzugt eine Kombination aus Phospholipiden und einem Kontaktaktivator aus der Gruppe Ellagsäure, Kaolin und Silica. Das Gerinnungsaktivator-Reagenz kann ferner Calciumchlorid enthalten. Alternativ kann das Calciumchlorid als separates Reagenz in dem Testkit enthalten sein.

In noch einer anderen Ausführungsform kann das Testkit ein Reagenz enthalten, das eine Mischung des Gerinnungsaktivators des intrinsischen Blutgerinnungssystems und der mindestens einen neutralisierenden Substanz enthält.

Noch ein weiterer Gegenstand der vorliegenden Erfindung ist die Verwendung eines erfindungsgemäßen Testkits in einem Verfahren zum Ausschluss von Antikoagulanzien aus der Gruppe der Faktor XIa-Inhibitoren in einer Patientenprobe, vorzugsweise in einem Verfahren wie weiter oben beschrieben.

Unabhängig vom grammatikalischen Geschlecht eines bestimmten Begriffes sind Personen mit männlicher, weiblicher oder anderer Geschlechteridentität umfasst.

### FIGURENBESCHREIBUNG

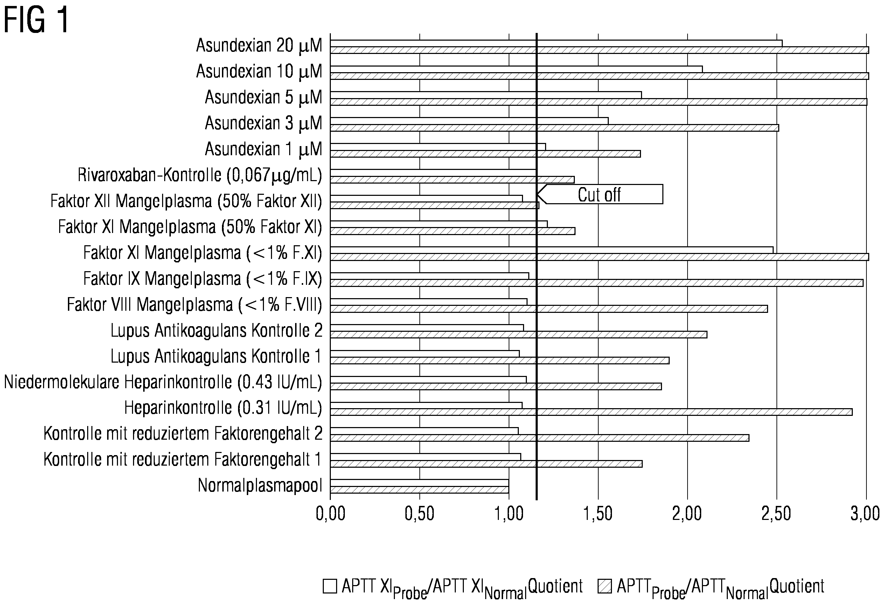

- FIG. 1: zeigt eine graphische Darstellung der für verschiedene Proben erfindungsgemäß ermittelten Quotienten APTT XI_{Probe}/APTT XI_{Normal}, die aus den erfindungsgemäß bestimmten APTT-Gerinnungszeiten einer Probe und einer Normalplasmaprobe berechnet wurden, im Vergleich zu den Quotienten APTT_{Probe}/APTT_{Normal}, die aus den herkömmlich bestimmten APTT-Gerinnungszeiten einer Probe und einer Normalplasmaprobe berechnet wurden. Für Proben mit einem Quotienten APTT XI_{Probe}/APTT XI_{Normal} unterhalb des Cut off-Werts von 1,21 kann die Präsenz von ≥ 1 µM Asundexian (therapeutischer Faktor XIa-Inhbitor) ausgeschlossen werden.

Die folgenden Beispiele dienen der Veranschaulichung und sind nicht als Einschränkung zu verstehen.

### BEISPIELE

- **BEISPIEL 1:**: **Bestimmung der herkömmlichen APTT und der erfindungsgemäßen, modifizierten APTT (APTT XI) in Proben mit unterschiedlichem Blutgerinnungsstatus**

Zur Aktivierung des intrinsischen Blutgerinnungssystems wurde ein APTT-Reagenz (Dade Actin FS Activated PTT Reagenz, Siemens Healthineers) enthaltend Ellagsäure und Phospholipide verwendet. Für die Bestimmung der herkömmlichen APTT wurden 50 µL einer (unverdünnten) Probe mit 50 µL des APTT-Reagenzes vermischt. Nach einer dreiminütigen Inkubation bei 37 °C wurden dem Reaktionsgemisch 50 µL einer 25 mM Calciumchloridlösung zugegeben, und es wurde die APTT-Gerinnungszeit in Sekunden auf einem automatischen Analysegerät bestimmt.

Für die Durchführung des erfindungsgemäßen Verfahrens wurde das besagte APTT-Reagenz so modifiziert, dass es neben der Ellagsäure und den Phospholipiden zusätzlich die Heparin-neutralisierende Substanz Protaminsulfat (2 ug/mL) enthielt. Ferner wurden die Proben vor Zugabe des modifizierten APTT-Reagenzes mit Faktor XI-Mangelplasma vermischt, indem ein Volumenteil Probe mit 2 Volumenteilen Faktor XI-Mangelplasma vermischt wurden. Für die Bestimmung der erfindungsgemäßen, modifizierten APTT XI wurden 50 µL einer mit Faktor XI-Mangelplasma vermischten Probe mit 50 µL des modifizierten APTT-Reagenzes vermischt. Nach einer dreiminütigen Inkubation bei 37 °C wurden dem Reaktionsgemisch 50 µL einer 25 mM Calciumchloridlösung zugegeben, und es wurde die APTT XI-Gerinnungszeit in Sekunden auf einem automatischen Analysegerät bestimmt.

Es wurden Plasmaproben mit verschiedenen Gerinnungsstörungen getestet, die bekanntermaßen eine gegenüber dem Normal verlängerte APTT-Gerinnungszeit aufweisen, z.B. Proben mit Gerinnungsfaktormängeln, Plasmaproben enthaltend verschiedene Antikoagulanzien oder enthaltend andere Störfaktoren (z.B. Lupus anticoagulans) (siehe Tabelle 1).

Insbesondere wurden folgende Probentypen getestet:

| **Probentyp** | **Beschreibung** |
|---|---|
| Normalplasmapool | Plasmapool von > 20 gesunden Spendern; |
| Asundexian | Normalplasma gespikt mit unterschiedlichen Mengen Asundexian (Faktor XIa-Inhibitor); |
| Rivaroxaban-Kontrolle | Normalplasma gespikt mit Rivaroxaban (Faktor Xa-Inhibitor); |
| Faktor VIII, IX, XI oder XII Mangelplasma | Faktor VIII, IX, XI oder XII depletierte Plasmen (< 1 % Faktorgehalt) bzw. Mischungen eines depletierten Plasmas mit Normalplasma; |
| Niedermolekulare Heparinkontrolle | Normalplasma gespikt mit niedermolekularem Heparin; |
| Heparinkontrolle | Normalplasma gespikt mit unfraktioniertem Heparin; |
| Kontrolle mit reduziertem Faktorengehalt | Plasmapool mit verminderter Aktivität intrinsischer Gerinnungsfaktoren (Faktor VIII, IX, XI oder XII); |
| Lupus Anticoagulans Kontrolle | Einzelspenderplasmen, Lupus anticoagulans-positiv in einem auf |
| | dilute Russel's Viper Venom (DRVVT)-Test beruhenden Testsystem. |

Für jede Probe wurden die herkömmliche APTT (APTT) und die erfindungsgemäße, modifizierte APTT (APTT XI) in Sekunden bestimmt, und es wurde für jede Probe 1.) der Quotient der für die Probe bestimmten APTT und der APTT eines Normalplasmapools (APTT_{Probe}/APTT_{Normal}) und 2.) der Quotient der für die Probe bestimmten APTT XI und der APTT XI des Normalplasmapools (APTT XI_{Probe}/APTT XI_{Normal}) berechnet (siehe Tabelle 1). Die Ergebnisse sind auch in Figur 1 graphisch dargestellt.

**Tabelle 1**

| **Probe** | **APTT [s]** | **APTT XI [s]** | **APTT_{Probe}/ APTT_{Normal}** | **APTT XI probe/ APTT XI_{Normal}** |
|---|---|---|---|---|
| Normalplasma | 28.19 | 44.7 | 1.00 | 1.00 |
| Asundexian 1 µM | 48.95 | 53.89 | 1.74 | 1.21 |
| Asundexian 3 µM | 70.74 | 69.52 | 2.51 | 1.56 |
| Asundexian 5 µM | 84. 67 | 77.89 | 3.00 | 1.74 |
| Asundexian 10 µM | 106.7 | 93.09 | 3.79 | 2.08 |
| Asundexian 20 µM | 130.8 | 113.9 | 4.64 | 2.53 |
| Rivaroxaban-Kontrolle 67 ng/mL | 38.5 | 51.6 | 1.37 | 1.15 |
| Faktor VIII Mangelplasma (< 1% F.VIII) | 68.99 | 49.22 | 2.45 | 1.10 |
| Faktor IX Mangelplasma (< 1% F.IX) | 84.1 | 49. 63 | 2.98 | 1.11 |
| Faktor XI Mangelplasma (< 1% F.XI) | 119.2 | 110.8 | 4.23 | 2.48 |
| Faktor XI Mangelplasma (< 50% F.XI) | 38.6 | 54.3 | 1.37 | 1.21 |

| **Probe** | **APTT [s]** | **APTT XI [s]** | **APTT_{Probe}/ APTT_{Normal}** | **APTT XI_{Probe/} APTT XI_{Normal}** |
|---|---|---|---|---|
| Faktor XII Mangelplasma (< 50% F.XII) | 32.9 | 48.1 | 1.17 | 1.08 |
| Niedermolekulare Heparinkontrolle (0.43 IU/mL) | 82.35 | 47. 97 | 1.85 | 1.10 |
| Heparinkontrolle (0.31 IU/mL) | 52.26 | 49.01 | 1.90 | 1.07 |
| Lupus Anticoagulans Kontrolle 1 (niedrig positiv) | 53.44 | 47.32 | 1.90 | 1.06 |
| Lupus Anticoagulans Kontrolle 2 (hoch positiv) | 59.41 | 48.35 | 2.11 | 1.08 |
| Kontrolle mit reduziertem Faktorengehalt 1 | 49.23 | 47.61 | 1.75 | 1.05 |
| Kontrolle mit reduziertem Faktorengehalt 2 | 66.05 | 47 | 2.34 | 1.07 |

Die Ergebnisse zeigen, dass ein erfindungsgemäß bestimmter Quotient APTT XI_{Probe}/APTT XI_{Normal} < 1,21 in dem getesteten Probenkollektiv die Präsenz von ≥ 1 µM Asundexian (therapeutischer Faktor XIa-Inhbitor) in der Probe ausschließt. Lediglich Proben mit starkem Faktor XI-Mangel (≤ 50 %) können nicht sicher ausgeschlossen werden; aufgrund der Seltenheit dieser Erkrankung ist der Effekt auf die Spezifität des Verfahrens in der Praxis jedoch vernachlässigbar. Der Quotient APTT_{Probe}/APTT_{Normal}, der aus den herkömmlich bestimmten APTT-Gerinnungszeiten einer Probe und einer Normalplasmaprobe berechnet wird, eignet sich hingegen nicht zur Differenzierung von Faktor XIa-Inhibitor positiven und negativen Proben.

Der Ausschluss von Faktor XIa-Inhibitoren wird mit dem hier ermittelten Cut off-Wert von 1,21 in großen Patientenkollektiven voraussichtlich nicht vollständig selektiv sein. Der bestmögliche Cut off-Wert muss -wie in solchen Fällen üblich- in einer klinischen Studie mit einer statistisch hinreichenden Anzahl von Patientenproben und mit Hilfe einer Receiver Operator Characteristic Analyse bestimmt werden, wobei zwischen Sensitivität und Spezifität abzuwägen ist.

## Patentansprüche

1. Verfahren zum Ausschluss von Antikoagulanzien aus der Gruppe der Faktor XIa-Inhibitoren in einer Patientenprobe, das Verfahren umfassend die Schritte
a) Herstellen eines ersten Reaktionsgemisches enthaltend die Patientenprobe und eines zweiten Reaktionsgemisches enthaltend eine Normalplasmaprobe durch Vermischen jeder der beiden Proben mit
i) einem Faktor XI-Mangelplasma, und
ii) mindestens einer neutralisierenden Substanz, die ein anderes Antikoagulanz als ein Antikoagulanz aus der Gruppe der Faktor XIa-Inhibitoren neutralisiert, und
iii) einem Gerinnungsaktivator des intrinsischen Blutgerinnungssystems;
b) Messen der Gerinnungszeit in dem ersten Reaktionsgemisch (APTT XI_{Probe}) und der Gerinnungszeit in dem zweiten Reaktionsgemisch (APTT XI_{Normal});
c) Bilden des Quotienten APTT XI_{Probe}/APTT XI_{Normal} aus beiden Gerinnungszeiten;
d) Vergleichen des Quotienten mit einem vorbestimmten Referenzwert; und
e) Ausschließen von Antikoagulanzien aus der Gruppe der Faktor XIa-Inhibitoren in der Patientenprobe, wenn der Quotient APTT XI_{Probe}/APTT XI_{Normal} unterhalb des vorbestimmten Referenzwerts liegt.

2. Verfahren gemäß Anspruch 1, wobei die mindestens eine neutralisierende Substanz ein Antikoagulanz aus der Gruppe der Thrombin- und FXa-Inhibitoren neutralisiert.

3. Verfahren gemäß Anspruch 2, wobei die mindestens eine ein Antikoagulanz aus der Gruppe der Thrombin-Inhibitoren neutralisierende Substanz Idarucizumab ist und die mindestens eine ein Antikoagulanz aus der Gruppe der FXa-Inhibitoren neutralisierende Substanz Andexanet alfa oder Ciraparantag ist.

4. Verfahren gemäß Anspruch 2, wobei die mindestens eine neutralisierende Substanz Heparin neutralisiert.

5. Verfahren gemäß Anspruch 4, wobei die mindestens eine Heparin-neutralisierende Substanz Protaminsulfat ist.

6. Verfahren gemäß einem der vorhergehenden Ansprüche zum Ausschluss eines Faktor XIa-Inhibitors aus der Gruppe Asundexian, Milvexian, Abelacimab, Osocimab, Gruticibart (Xisomab), REGN9933, MK-2060, ONO-7684 und ONO-5450598.

7. Testkit zur Verwendung in einem Verfahren zum Ausschluss von Antikoagulanzien aus der Gruppe der Faktor XIa-Inhibitoren in einer Patientenprobe, das Testkit
enthaltend
a) ein Faktor XI-Mangelplasma, und
b) mindestens eine neutralisierende Substanz, die ein anderes Antikoagulanz als ein Antikoagulanz aus der Gruppe der Faktor XIa-Inhibitoren neutralisiert.

8. Testkit gemäß Anspruch 7, wobei die mindestens eine neutralisierende Substanz ein Antikoagulanz aus der Gruppe der Thrombin- und FXa-Inhibitoren neutralisiert.

9. Testkit gemäß Anspruch 8, wobei die mindestens eine ein Antikoagulanz aus der Gruppe der Thrombin-Inhibitoren neutralisierende Substanz Idarucizumab ist und die mindestens eine ein Antikoagulanz aus der Gruppe der FXa-Inhibitoren neutralisierende Substanz Andexanet alfa oder Ciraparantag ist.

10. Testkit gemäß Anspruch 8, wobei die mindestens eine neutralisierende Substanz Heparin neutralisiert.

11. Testkit gemäß Anspruch 10, wobei die mindestens eine neutralisierende Substanz Protaminsulfat ist.

12. Testkit gemäß einem der Ansprüche 7 bis 11, ferner enthaltend einen Gerinnungsaktivator des intrinsischen Blutgerinnungssystems.

13. Testkit gemäß Anspruch 12, wobei der Gerinnungsaktivator des intrinsischen Blutgerinnungssystems eine Kombination aus Phospholipiden und einem Kontaktaktivator, bevorzugt eine Kombination aus Phospholipiden und einem Kontaktaktivator aus der Gruppe Ellagsäure, Kaolin und Silica, ist.

14. Testkit gemäß einem der Ansprüche 7 bis 13, ferner enthaltend Calciumchlorid.

15. Testkit gemäß einem der Ansprüche 7 bis 14, wobei das Testkit ein Reagenz enthält, das eine Mischung des Faktor XI-Mangelplasmas und der mindestens einen neutralisierenden Substanz enthält.

16. Testkit gemäß einem der Ansprüche 12 bis 14, wobei das Testkit ein Reagenz enthält, das eine Mischung des Gerinnungsaktivators des intrinsischen Blutgerinnungssystems und der mindestens einen neutralisierenden Substanz enthält.

17. Verwendung eines Testkits gemäß einem der Ansprüche 7 bis 16 in einem Verfahren zum Ausschluss von Antikoagulanzien aus der Gruppe der Faktor XIa-Inhibitoren in einer Patientenprobe.
